# EUROPEAN PATENT APPLICATION

(11) **EP 3 438 249 A1**
(43) Date of publication of application: **06.02.2019**
(21) Application number: 17774717.7
(22) Date of filing: 23.03.2017
(51) Int. Cl.: C12N 5/0797

(54) **MEDIUM FOR NEURAL STEM CELLS ENHANCING NEURAL DIFFERENTIATION ABILITY**

(30) Priority: 31.03.2016 JP 2016071967
(71) Applicant: Ajinomoto Co., Inc., Tokyo 104-8315 (JP)
(72) Inventor: MATSUMOTO, Takuya, Kawasaki-shi Kanagawa 210-8681 (JP); HARATA, Ikue, Kawasaki-shi Kanagawa 210-8681 (JP); SENDA, Sho, Kawasaki-shi Kanagawa 210-8681 (JP)
(74) Representative: Hoffmann Eitle
(86) International application number: PCT/JP2017/011870
(87) International publication number: WO 2017/170180

(57) **Abstract**

The present invention provides a medium for neural stem cells and/or neural progenitor cells and substantially free of one or more amino acids selected from the group consisting of L-glutamine, L-asparagine and L-aspartic acid, and a method for culturing the neural stem cells and/or neural progenitor cells in the medium, and the like.

## Description

### [Technical Field]

The present invention relates to a culture medium and a culture method for neural stem cells and/or neural progenitor cells. More particularly, the present invention relates to a medium promoting the neuronal differentiation potency of neural stem cells and/or neural progenitor cells, and a method for promoting the neuronal differentiation potency of neural stem cells and/or neural progenitor cells by using the medium and the like.

### [Background Art]

As one of the methods for treating refractory neurological diseases and nerve damages such as amyotrophic lateral sclerosis, Alzheimer's disease, Parkinson's disease and the like, the development of a method of transplanting nerve cells and neural stem cells to the living body has been expected in recent years. Neural stem cell is an undifferentiated cell having self-replication competence and multipotency and the ability to produce various cells of the nerve system (nerve cell and neural progenitor cell (neural progenitor), as well as glial cells (astrocyte, oligodendrocyte etc.) and glial progenitor cell etc.). In addition to transplantation of themselves, neural stem cells and neural progenitor cells can supply cells that are difficult to proliferate in normal adults such as nerve cells and the like. Therefore, they are attracting attention as a source of biomaterials in regenerative medicine.

Neural stem cells have proliferation potency and multipotency, and they are thus considered to produce functional neuronal cells in vivo when transplanted into a living body. On the other hand, tumorigenesis by transplanting neural stem cells with proliferation potency is feared. Therefore, the use of neural progenitor cells differentiated in advance from neural stem cells in vitro for regenerative medicine has been studied. Even in this case, there is a risk that the prepared neural progenitor cells are contaminated with cells having proliferation potency and the cells remain in the progenitor cells. To avoid formation of tumor from contamination of neuronal cells after transplantation with proliferative cells, it is important to not contain proliferative cells as much as possible. When a treatment with nonproliferative nerve cells is performed, it is important that neural stem cells can differentiate highly efficiently into nerve cells. Therefore, the development of a method for producing neuronal cells highly efficiently from neural stem cells or the development of a method for preparing neuronal cells while reducing proliferative cells contained in the obtained differentiated neuronal cells is the important problem.

In the meantime, there are several reports on a medium not containing glutamine.

Non-patent document 1 describes that differentiation of human erythroleukaemia cell line K562 cells (hematopoietic cells) into erythrocytes is promoted by culturing in a glutamine-free medium.

Non-patent document 2 describes that differentiation of C6 glioma cells (rat glioma cells) into oligodendrocyte-like cells is promoted by culturing in a glutamine-free medium.

However, it has not been clarified at all what kind of influence the medium not containing one or more amino acids from glutamine, asparagine and aspartic acid has on the differentiation potency and nerve cell producibility of neural stem cells and/or neural progenitor cells.

### [Document List]

### [non-patent documents]

non-patent document 1: Canh Hiep N. et al., "Depletion of glutamine enhances sodium butyrate-induced erythroid differentiation of K562 cells." The Journal of Biochemistry. 2012 Dec; 152(6):509-519.
non-patent document 2: Martin M. et al., "Energetic and morphological plasticity of C6 glioma cells grown on 3-D support; effect of transient glutamine deprivation." Journal of Bioenergetics and Biomembranes. 1998 Dec; 30(6):565-78.

### [SUMMARY OF THE INVENTION]

### [Problems to be Solved by the Invention]

An object of the present invention is to provide a medium capable of promoting the neuronal differentiation potency of neural stem cells and/or neural progenitor cells, and a method for promoting the neuronal differentiation potency of neural stem cells and/or neural progenitor cells by using the medium.

### [Means of Solving the Problems]

The present inventors have conducted intensive studies in an attempt to achieve the above-mentioned object and found that the proliferation potency of neural stem cells can be suppressed and the neuronal differentiation potency of the cells is promoted by culturing the cells in a medium from which one or more amino acids selected from the group consisting of L-glutamine, L-asparagine and L-aspartic acid has/have been removed. It was possible to passage neural stem cells while maintaining promoted neuronal differentiation potency by using a medium from which one or more amino acids selected from the group consisting of L-glutamine, L-asparagine and L-aspartic acid was/were removed. When neural stem cells with promoted neuronal differentiation potency were differentiated into nerve cells, a nerve cell population with low proliferation property and a high proportion of differentiated cells was obtained. The present inventors have conducted further investigation based on these findings and completed the present invention.

Therefore, the present invention provides the following.
(1) A medium substantially free of L-glutamine, for culturing neural stem cells and/or neural progenitor cells.
(2) The medium of (1), having an L-glutamine concentration of not more than 100 µM.
(3) The medium of (1) or (2), not comprising L-glutamine.
(4) The medium of any of (1) to (3), comprising holotransferrin.
(5) The medium of any of (1) to (4), comprising L-tryptophan, L-lysine, L-methionine, L-phenylalanine, L-threonine, L-valine, L-leucine, L-isoleucine and L-histidine.
(6) The medium of any of (1) to (5) for promoting the neuronal differentiation potency of a neural stem cell and/or a neural progenitor cell.
(7) The medium of any of (1) to (6) that is a serum-free medium.
(8) A method for culturing a neural stem cell and/or a neural progenitor cell, comprising culturing the cell(s) in the medium of any of (1) to (7).
(9) The method of (8) for producing a neural stem cell and/or a neural progenitor cell with promoted neuronal differentiation potency.
(10) The method of (8) or (9), wherein the culturing is performed for a period of not less than 4 days.
(11) A method for producing a nerve cell, comprising the following steps:
   (I) culturing a neural stem cell and/or a neural progenitor cell in the medium of any of (1) to (7) to give the neural stem cell and/or the neural progenitor cell with promoted neuronal differentiation potency;
   (II) culturing the neural stem cell and/or the neural progenitor cell with promoted neuronal differentiation potency and obtained in step (I) under nerve cell differentiation induction conditions to give a nerve cell population.
(12) A neural stem cell and/or a neural progenitor cell with promoted neuronal differentiation potency and obtained by the method of (9).
(13) A culture preparation comprising a neural stem cell and/or a neural progenitor cell, and the medium of any of (1) to (7).

### [Effect of the Invention]

The medium of the present invention has an effect of promoting the neuronal differentiation potency of neural stem cells and/or neural progenitor cells. When neural stem cells and/or neural progenitor cells are cultured by the culture method of the present invention, neural stem cells and/or neural progenitor cells having suppressed proliferation potency and promoted neuronal differentiation potency can be obtained. Neural stem cells and/or neural progenitor cells with promoted neuronal differentiation potency generate many nerve cells by differentiation induction culture, and therefore, efficiently nerve cells can be produced. Since the nerve cell population thus produced contains few proliferative cells, the risk of tumorigenesis when the nerve cells are transplanted into a living body is reduced, and the nerve cell population can contribute to the development of a safer treatment method.

### [Brief Description of the Drawings]

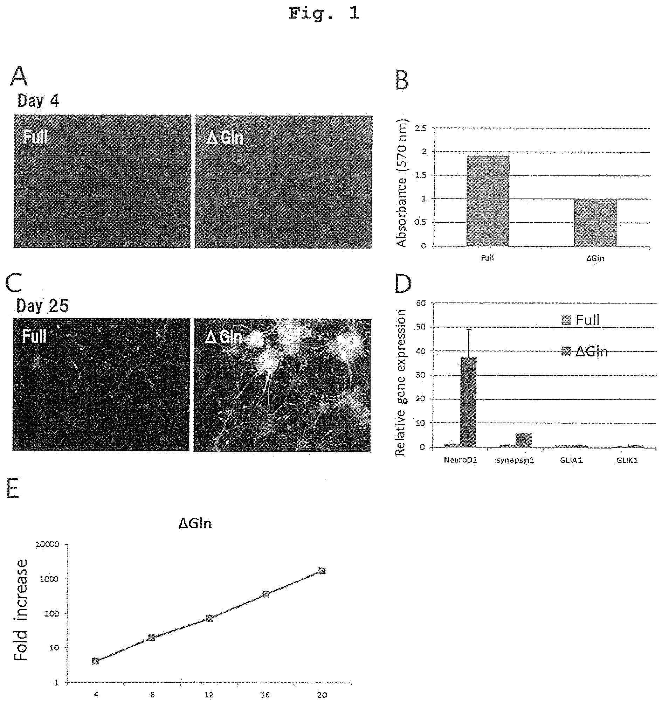

Fig. 1 shows a neuronal differentiation promoting effect by an L-glutamine-removed medium. Neural stem cells were cultured for 4 days (Day4) in a medium containing L-glutamine (Full) or a medium not containing L-glutamine (ΔGln) (Fig. 1A and B). The neural stem cells cultured in the medium not containing L-glutamine showed decreased proliferation property of the neural stem cells (Fig. 1B). After culturing in the above-mentioned medium for 4 days, the medium was exchanged with a neuronal differentiation induction medium, the cells were cultured for 21 days (Day25), and the nerve cells were immunostained. The neural stem cells cultured in the medium not containing L-glutamine (ΔGln) generated many nerve cells as compared to the medium containing L-glutamine (Full) (Fig. 1C). Fig. 1D shows the expression intensity of NeuroD1, Synapsin1. In Fig. 1D, the left bar of each item shows Full and the right bar shows ΔGln. The proliferation curve of the neural stem cells cultured in the medium not containing L-glutamine for 20 days showed linearity (Fig. 1E).
Fig. 2 shows the analysis results of the intracellular amino acid content. Horizontal axis: quantified amino acid, vertical axis: amino acid content per cell number (nmol/1x10⁶ cells). The left bar of each item shows Full and the right bar shows ΔGln.
Fig. 3 shows an organic acid content in TCA cycle per cell number. The left bar of each item shows Full and the right bar shows ΔGln.
Fig. 4 shows apoptosis in neural stem cells.

### [Description of Embodiments]

The present invention provides a medium for culturing neural stem cells and/or neural progenitor cells (hereinafter these are also generically referred to as the medium of the present invention), a method for culturing the neural stem cells and/or neural progenitor cells in the medium (hereinafter these are also generically referred to as the culture method of the present invention), and neural stem cells and/or neural progenitor cells having promoted neuronal differentiation potency obtained by the method, and the like.

### (1) Neural stem cells and/or neural progenitor cells

In the present specification, the neural stem cell means an undifferentiated cell having self-replication competence and maintaining multipotency into neuronal cells (nerve cell and glial cell (astrocyte, oligodendrocyte and the like), and progenitor cells thereof). Specifically, the neural stem cell is a cell having potency to finally generate nerve cells and glial cells (astrocyte, oligodendrocyte and the like) and not substantially generating cells other than nerve system cells such as epidermal cells, hematopoietic cells, myocytes and the like unless a special operation is applied such as reprogramming and the like. Not substantially generating refers to a state where not less than 90% of the cells generated by neural stem cells are any of nerve cells and glial cells (astrocytes, oligodendrocytes and the like), and progenitor cells thereof.

In the present specification, the neural progenitor cell (neural progenitor) refers to an undifferentiated cell having division potential and having an ability to finally differentiate into one or more kinds of nerve cells. The neural progenitor cell is fate determined to finally generate nerve cells and not substantially generate cells other than nerve cells and progenitor cells thereof. The glial progenitor cell (glial progenitor) is an undifferentiated cell derived from neural stem cells and having division potential. It has an ability to finally differentiate into any of astrocyte, oligodendrocyte, microglia, ependymal cell and Schwann cell, or a progenitor cell thereof, and not substantially differentiate into nerve cell.

It is difficult to strictly distinguish neural stem cell and neural progenitor cell from each other. In the present specification, therefore, they are sometimes used as "neural stem cells and/or neural progenitor cells" without distinction.

In the present invention, neural stem cells and/or neural progenitor cells derived from a mammal are generally used. Examples of the mammal include, but are not limited to, rodents such as mouse, rat, hamster, guinea pig and the like, lagomorphs such as rabbit and the like, ungulates such as swine, bovine, goat, horse, sheep and the like, carnivoras such as dog, cat and the like, primates such as human, monkey, Macaca mulatta, marmoset, orangutan, chimpanzee and the like, and the like. The neural stem cells and/or neural progenitor cells to be used in the present invention are preferably neural stem cells and/or neural progenitor cells of rodents such as mouse and the like or primates such as human and the like, more preferably, neural stem cells and/or neural progenitor cells of human.

The neural stem cells and/or neural progenitor cells to be used in the present invention include those derived from pluripotent stem cell, separated from biological tissue, obtained by directly inducing differentiation from fibroblasts and the like without intervention of pluripotent stem cells (Matsui T et al., Stem Cells. 2012 Jun; 30(6):1109-19) and the like, and are not particularly limited as long as they maintain the undifferentiated state described above, maintain multipotency and maintain potency to generate nerve cells. The neural stem cells and/or neural progenitor cells to be used in the present invention are preferably derived from pluripotent stem cells, more preferably derived from induced pluripotent stem cells (iPS cells).

As a method for producing neural stem cells and/or neural progenitor cells from pluripotent stem cells, methods known per se can be used. Examples of the production method neural stem cells and/or neural progenitor cells from pluripotent stem cells include, but are not limited to, a method for forming neural stem cells and/or neural progenitor cells via embryoid body formation by performing suspension culture of pluripotent stem cells (Bain G et al., Dev Biol. 1995 Apr; 168(2):342-57 and the like), a method using stromal cells and the like as feeder cells, a method including suspension culturing pluripotent stem cells in a serum-free medium containing bFGF (Watanabe K et al., Nat Neurosci. 2005 Mar; 8(3):288-96 and the like), a method including adhesion culturing pluripotent stem cells (ES cells etc.) in the presence of SMAD signal inhibitors Noggin and SB431542 (Chambers SM et al., Nat Biotechnol. 2009 Mar; 27(3):275-80), a method including culturing a single layer-cultured pluripotent stem cells (ES cells etc.) in the presence of glycogen synthase kinase 3 (GSK3) inhibitor, transforming growth factor β (TGF-β) inhibitor, Notch signal inhibitor (Li W et al., Proc Natl Acad Sci USA. 2011 May 17; 108(20):8299-304) and the like.

In the present specification, pluripotent stem cell means an immature cell having self-replication competence and differentiation/proliferation potency, and having potency to differentiate into any tissues or cells constituting the living body except placenta. Examples of the pluripotent stem cell include embryonic stem cell (ES cell), induced pluripotent stem cell (iPS cell) (Takahashi K et al., Cell. 2007 Nov 30; 131(5):861-72), spermatozoon stem cell (mGS cell) (Kanatsu-Shinohara M et al., Biol Reprod. 2007 Jan; 76(1):55-62), embryonic reproductive cell (Matsui Y et al., Cell. 1992 Sep 4; 70(5):841-7) and the like.

That the cell is a neural stem cell can be confirmed by, for example, suspension culturing the cell in a serum-free medium containing EGF and bFGF, dispersion treating the cultured cell aggregates, adhesion culturing them in the differentiation induction medium described in the below-mentioned Examples, and confirming with the production of both nerve cells and glial cells as an index.

The neural stem cell can also be confirmed by a gene known to express in neural stem cells, a transcription product, a protein and the like thereof (neural stem cell markers).

Examples of known neural stem cell markers include a cell skeleton protein Nestin; Science, 276, 66(1997), SOX1 (SRY (sex determining region Y)-box1), SOX2 (SRY (sex determining region Y)-box2), Pax6 (paired box 6), Ki67, proliferation cell nuclear antigen (PCNA), fatty acid binding protein 7 (to be also referred to as Fabp7, aka BLBP) and the like, and those of ordinary skill in the art can confirm a desired neural stem cell by appropriately combining these markers.

That a cell is a neural progenitor cell can be confirmed by, for example, suspension culturing the cell in a serum-free medium containing EGF and bFGF, dispersion treating the cultured cell aggregates, adhesion culturing them in the differentiation induction medium described in the below-mentioned Examples, and confirming with the production of nerve cell and no production of glial cell as an index.

In addition, the neural progenitor cell can also be confirmed by a gene known to express in neural progenitor cell, a transcription product, a protein and the like thereof (neural progenitor cell markers). Examples of the genes expressed in neural progenitor cells include, but are not limited to, Tbr2, MASH1, Nestin, NeuroD1 and the like. Those of ordinary skill in the art can confirm that the cell is a neural progenitor cell by appropriately combining these markers.

Examples of the neural progenitor cell include, but are not limited to, SOX2 negative and Nestin-positive cells.

In the present specification, nerve cell (neuron) means a cell to be the functional unit of the nerve system. Generally, nerve cell is constituted of cell body and neurite (e.g., axon, dendrite), and nerve cells without neurite (non-polar nerve cell) such as juvenile neuron in the developing stage, amacrine cell of retina, granule cell of olfactory bulb and the like are also present. When nerve cells are classified by forms, they are classified into non-polar nerve cell, unipolar nerve cell, pseudounipolar nerve cell, bipolarnerve cell and multipolar nerve cell. In the present specification, the nerve cell includes all of these.

The nerve cell encompasses central nervous system nerve cells and peripheral nervous system nerve cells. The nerve cell may be a motoneuron cell or a sensory nerve cell or an interneuron. Examples of the nerve cell include, but are not limited to, dopaminergic nerve cell, noradrenergic nerve cell, adrenergic nerve cell, serotonergic nerve cell, cholinergic nerve cell, gamma-aminobutyric acidergic nerve cell, glutamatergic nerve cell and the like.

That the cell is a nerve cell can be confirmed by a gene known to express in nerve cell, a transcription product, a protein and the like thereof (nerve cell markers).

Examples of the markers of differentiated nerve cell include MAP2 (microtubule associated protein 2), neurofilament, synapsin 1, βIII tubulin, NeuN (neuronal specific nuclear protein), FOX3 (Forkhead box protein), calbindin, PSA-NCAM (polysialylated neural cell adhesion molecule), Doublecortin, Peripherin and the like.

Whether the cells produced by the neural stem cells and/or neural progenitor cells are nerve cells can be confirmed by, for example, the aforementioned markers.

### (2) Medium for culturing neural stem cells and/or neural progenitor cells

The medium of the present invention is characterized in that it is substantially free of one or more amino acids selected from the group consisting of L-glutamine (2-amino-4-carbamoylbutyric acid), L-asparagine (2-amino-3-carbamoylpropionic acid) and L-aspartic acid (2-aminobutanedioic acid) (to be also referred to as "neuronal differentiation associated amino acid" in the present specification).

The "neuronal differentiation associated amino acid" to be selected may be one kind (L-glutamine, L-asparagine, or L-aspartic acid) (in which case the medium of the present invention may contain other two kinds of neuronal differentiation associated amino acids), two kinds (combination of L-glutamine and L-asparagine, combination of L-glutamine and L-aspartic acid, or combination of L-asparagine and L-aspartic acid) (in which case the medium may contain another kind of neuronal differentiation associated amino acid), or all three kinds (L-glutamine, L-asparagine and L-aspartic acid).

In one embodiment, the present invention provides a medium characterized in that it is substantially free of amino acid selected from the group consisting of L-glutamine, L-asparagine and L-aspartic acid. In the embodiment, the medium of the present invention may contain one or both of the other two kinds of neuronal differentiation associated amino acids not selected.

The medium of the present invention may be a medium for promoting the neuronal differentiation potency of neural stem cells and/or neural progenitor cells. The neuronal differentiation potency of neural stem cells and/or neural progenitor cells can be promoted by culturing the cells in the medium of the present invention. Furthermore, when the neural stem cells and/or neural progenitor cells cultured in the medium of the present invention are differentiated into nerve cells, the number of proliferative cells in the obtained nerve cell population decreases. Therefore, it is possible to provide nerve cells useful for a nerve cell transplantation treatment, and neural stem cells and/or neural progenitor cells suitable for producing nerve cells for a nerve cell transplantation treatment.

In the present specification, the "medium is substantially free of one or more amino acids selected from the group consisting of L-glutamine, L-asparagine and L-aspartic acid" means that, when the neural stem cells and/or neural progenitor cells are cultured in the medium, the concentration of the selected neuronal differentiation associated amino acid in the medium is decreased to a level sufficient for promoting neuronal differentiation potency of the neural stem cells and/or neural progenitor cells as compared to culturing in a control medium having the same composition except that the selected neuronal differentiation associated amino acid is contained at an amino acid concentration corresponding to that of the standard medium (L-glutamine 0.3-3.0 mM (e.g., 2.5 mM); L-asparagine 0.05-1.0 mM (e.g., 0.05 mM); L-aspartic acid 0.05-1.0 mM (e.g., 0.05 mM)).

For example, the "medium is substantially free of L-glutamine" means that, when the neural stem cells and/or neural progenitor cells are cultured in the medium, the concentration of the L-glutamine in the medium is decreased to a level sufficient for promoting neuronal differentiation potency of the neural stem cells and/or neural progenitor cells as compared to culturing in a control medium having the same composition except that the L-glutamine is contained at an L-glutamine concentration corresponding to that of the standard medium (0.3-3.0 mM (e.g., 2.5 mM)). The same applies to other neuronal differentiation associated amino acids.

In the present specification, being "substantially free of one or more amino acids selected from the group consisting of L-glutamine, L-asparagine and L-aspartic acid" means that not only the selected neuronal differentiation associated amino acid but also a substance providing the selected neuronal differentiation associated amino acid such as alternative dipeptides of the selected neuronal differentiation associated amino acid (dipeptide containing the selected neuronal differentiation associated amino acid residue and supplying the selected neuronal differentiation associated amino acid by being decomposed by peptidase and the like in the medium) and the like are not contained in the medium.

In the present specification, being "substantially free of L-glutamine" means that the medium is substantially free of not only L-glutamine but also dipeptide of L-glutamine and an amino acid (L-glutamine alternative dipeptide) such as L-alanyl L-glutamine and the like.

In the present specification, being "substantially free of L-asparagine" means that the medium is substantially free of not only L-asparagine but also alternative dipeptide of L-asparagine (e.g., L-alanyl L-asparagine).

In the present specification, being "substantially free of L-aspartic acid" means that the medium is substantially free of not only L-aspartic acid but also alternative dipeptide of L-aspartic acid (e.g., L-alanyl L-aspartic acid).

The "medium substantially free of L-glutamine" means that the L-glutamine concentration (total concentration of L-glutamine and L-glutamine alternative dipeptide) of the medium is generally not more than 100 µM, preferably not more than 10 µM, more preferably not more than 100 nM, most preferably 0 nM.

The "medium substantially free of L-asparagine" means that the L-asparagine concentration (total concentration of L-asparagine and L-asparagine alternative dipeptide) of the medium is generally not more than 10 µM, preferably not more than 100 nM, more preferably not more than 1 nM, most preferably 0 nM.

The "medium substantially free of L-aspartic acid" means that the L-aspartic acid concentration (total concentration of L-aspartic acid and L-aspartic acid alternative dipeptide) of the medium is generally not more than 10 µM, preferably not more than 100 nM, more preferably not more than 1 nM, most preferably 0 nM.

In the present specification, not comprising a selected neuronal differentiation-associated amino acid refers to that the concentration of the neuronal differentiation-associated amino acid is 0 nM.

In the present specification, the "neuronal differentiation potency" means potency of neural stem cells and/or neural progenitor cells to differentiate into nerve cells.

The medium of the present invention has an effect of promoting the neuronal differentiation potency of neural stem cells and/or neural progenitor cells.

The "promoting the neuronal differentiation potency of neural stem cells and/or neural progenitor cells" means that, when the neural stem cells and/or neural progenitor cells cultured in the medium of the present invention, and the neural stem cells and/or neural progenitor cells cultured in the comparison control medium having the same composition as the medium of the present invention except that the concentration of the selected neuronal differentiation associated amino acid corresponds to that of the standard medium (L-glutamine 0.3-3.0 mM; asparagine 0.05-1.0 mM; aspartic acid 0.05-1.0 mM) (to be also referred simply to as comparison control cell in the present specification) are cultured under nerve cell differentiation conditions (e.g., in the presence of N2 and B27) to produce nerve cells, the number of nerve cells produced from a given cell number of the neural stem cells and/or neural progenitor cells is greater than that of the comparison control, or the number of nerve cells produced between the start of the culturing and a predetermined period is greater than that of the comparison control.

For example, when the selected neuronal differentiation associated amino acid is L-glutamine, the "promoting the neuronal differentiation potency of neural stem cells and/or neural progenitor cells" means that, when the neural stem cells and/or neural progenitor cells cultured in the medium of the present invention, and the neural stem cells and/or neural progenitor cells cultured in the comparison control medium having the same composition as the medium of the present invention except that the concentration of L-glutamine corresponds to that of the standard medium (0.3-3.0 mM (e.g., 2.5 mM)) are cultured under nerve cell differentiation conditions (e.g., in the presence of N2 and B27) to produce nerve cells, the number of nerve cells produced from one neural stem cell and/or neural progenitor cell is greater than that of the comparison control, or the number of nerve cells produced in a given period after the start of the culturing is greater than that of the comparison control.

In the medium of the present invention, components other than the selected neuronal differentiation associated amino acid are not particularly limited as long as they can promote neuronal differentiation potency of the neural stem cells and/or neural progenitor cells, and general compositions used for the maintenance culture of neural stem cells and/or neural progenitor cells can be appropriately adopted.

The medium of the present invention may be prepared as a basal medium having the composition of a medium generally used for culturing mammalian cells, from which the selected neuronal differentiation associated amino acid is removed. The basal medium is not particularly limited as long as a desired effect can be achieved. For example, media usable for culturing animal cells such as BME medium, BGJb medium, CMRL 1066 medium, Glasgow MEM medium, Improved MEM Zinc Option medium, IMDM medium, Medium 199 medium, Eagle MEM medium, αMEM medium, DMEM medium, F-12 medium, DMEM/F12 medium, IMDM/F12 medium, ham medium, RPMI 1640 medium, Fischer's medium, or a mixed medium of these, and the like can be mentioned. In addition, it can be prepared as a basal medium having the composition of a medium generally used for culturing pluripotent stem cells, from which the selected neuronal differentiation associated amino acid is removed.

It may be prepared as a basal medium having the compositions of StemFit (registered trade mark) AK medium (Ajinomoto Co., Inc.), Essential 8 medium (Life Technologies), mTeSR1 medium (STEMCELL Technologies), TeSR2 medium (STEMCELL Technologies), RHB medium (StemCells, Inc.), TeSR™-E6 (STEMCELL Technologies), hESF-GRO medium (NIPRO CORPORATION), HESF-DIF medium (NIPRO CORPORATION), CSTI-7 (Cell Science & Technology Institute, Inc.), Essential 6 medium (Life Technologies) and the like, which are commercially available basal media for culturing pluripotent stem cells, from which the selected neuronal differentiation associated amino acid is removed.

The medium of the present invention is preferably a medium containing components determined chemically (Chemically defined medium; CDM) to avoid contamination with chemically-undefined components.
The medium of the present invention is preferably a serum-free medium to avoid contamination with chemically-undefined components. The "serum-free medium" in the present invention means a medium free of an unadjusted or unpurified serum. In the present invention, a medium containing purified blood-derived components or animal tissue-derived components (e.g., growth factors such as bFGF and the like) is also included in the serum-free medium when an unadjusted or unpurified serum is absent.

The serum-free medium may contain a serum replacement. Examples of the serum replacement include those appropriately containing serum albumin, a fatty acid, collagen precursor, trace element, 2-mercaptoethanol or 3'thiolglycerol, or equivalents of these and the like. Such serum replacement can be prepared by the method described in, for example, WO 98/30679. As a serum replacement, a commercially available product may also be used. Examples of the commercially available serum replacement include, but are not limited to, Knockout™ Serum Replacement (manufactured by Life Technologies: hereinafter sometimes to be also indicated as KSR), Chemically-defined Lipid concentrated (manufactured by Life Technologies), B27 (Life Technologies Inc.), N2 (Life Technologies Inc.).

Generally, the medium of the present invention contains all essential amino acids (L-leucine, L-lysine, L-phenylalanine, L-isoleucine, L-threonine, L-histidine, L-methionine, L-tryptophan and L-valine).

While the concentration of the essential amino acids contained in the medium of the present invention is not particularly limited as long as the neuronal differentiation potency of neural stem cells and/or neural progenitor cells can be promoted, it is as follows:
L-leucine 0.005-100 mM, preferably 0.5-20 mM;
L-lysine 0.005-100 mM, preferably 1-20 mM;
L-phenylalanine 0.005-100 mM, preferably 0.5-10 mM;
L-isoleucine 0.005-100 mM, preferably 0.5-20 mM;
L-threonine 0.005-100 mM, preferably 0.5-10 mM;
L-histidine 0.005-100 mM, preferably 0.5-10 mM
L-methionine 0.005-100 mM, preferably 0.5-5 mM;
L-tryptophan 0.005-100 mM, preferably 0.05-1 mM;
L-valine 0.005-100 mM, preferably 0.5-10 mM.

Examples of the combination of the concentration of each essential amino acid include, but are not limited to, L-leucine 0.005-100 mM, L-lysine 0.005-100 mM, L-phenylalanine 0.005-100 mM, L-isoleucine 0.005-100 mM, L-threonine 0.005-100 mM, L-histidine 0.005-100 mM, L-methionine 0.005-100 mM, L-tryptophan 0.005-100 mM and L-valine 0.005-100 mM.

More preferably, the medium of the present invention contains all essential amino acids (L-leucine 0.5-20 mM, L-lysine 1-20 mM, L-phenylalanine 0.5-10 mM, L-isoleucine 0.5-20 mM, L-threonine 0.5-10 mM, L-histidine 0.5-10 mM, L-methionine 0.5-5 mM, L-tryptophan 0.05-1 mM and L-valine 0.5-10 mM).

The medium of the present invention preferably contains all non-essential amino acids other than L-glutamine, L-asparagine and L-aspartic acid (L-alanine 0.2-2 mM, L-arginine 1-10 mM, glycine 1-10 mM, L-glutamic acid 0.5-10 mM, L-cysteine 0.5-10 mM, L-serine 0.5-10 mM, L-tyrosine 0.5-10 mM, L-proline 0.5-5 mM).

When the medium of the present invention is substantially free of L-asparagine and/or L-aspartic acid and contains L-glutamine, the concentration of L-glutamine in the medium is preferably 0.3-3.0 mM.

When the medium of the present invention is substantially free of L-glutamine and/or L-aspartic acid and contains L-asparagine, the concentration of L-asparagine in the medium is preferably 0.05-1.0 mM.

When the medium of the present invention is substantially free of L-glutamine and/or L-asparagine and contains L-aspartic acid, the concentration of L-aspartic acid in the medium is preferably 0.05-1.0 mM.

The medium of the present invention may further contain natural amino acids such as L-cystine and the like, in addition to the aforementioned amino acids.

The medium of the present invention may further contain a medium additive. Examples of the medium additive include, but are not limited to, vitamins, proteins such as cytokines, growth factors and the like, L-ascorbic acid, ascorbyl-2-phosphate magnesium, sodium pyruvate, 2-aminoethanol(ethanolamine), glucose, sodium hydrogen carbonate, HEPES, insulin, progesterone, sodium selenate, putrescine and the like. The additives are preferably added at a concentration within the range known per se. The medium of the present invention may or may not contain L-ascorbic acid.

The medium of the present invention may further contain holotransferrin. Transferrin bound with iron is called holotransferrin, and transferrin not bound with iron is called apotransferrin. In the present specification, holotransferrin includes a molecule in which one apotransferrin molecule and one iron ion are bonded and a molecule in which one apotransferrin molecule and two iron ions are bonded.

When the medium of the present invention contains holotransferrin, the concentration of holotransferrin contained in the medium is not particularly limited as long as desired effects of promotion of neuronal differentiation potency of neural stem cells and/or neural progenitor cells and the like can be achieved, it is 0.01 - 100 µg/ml, preferably 0.1 - 6.5 µg/ml, further preferably 0.1 - 5 µg/ml.

As the nucleic acid sequence encoding the human transferrin, NM_001063 (NCBI Accession No.) can be mentioned and as the amino acid sequence of human transferring, NP_001054 can be mentioned, but they are not limited to these.

As holotransferrin, a commercially available reagent (Sigma Aldrich) can also be used.

The medium of the present invention may contain a growth factor used for passage culture of neural stem cells and/or neural progenitor cells (e.g., EGF: 10 - 100 ng/mL, bFGF: 10 - 100 ng/ml etc.).

The medium of the present invention may contain a fatty acid. Examples of the fatty acid to be contained in the medium of the present invention include, but are not limited to, oleic acid, linoleic acid, α-linolenic acid, γ-linolenic acid, palmitic acid, stearic acid, arachidonic acid, icosapentaenoic acid, docosahexaenoic acid, butyric acid, acetic acid, pulmitoleic acid, valeric acid, caproic acid, enanthic acid (heptylic acid), caprylic acid, pelargoric acid, capric acid, lauric acid, myristic acid, pentadecylic acid, margaric acid, khusenic acid, eleostearic acid, arachidic acid, 8,11-eicosadienoic acid, 5,8,11-eicosatrienoic, behenic acid, lignoceric acid, nervonic acid, cerotic acid, montanic acid, melissic acid and the like can be mentioned. The fatty acid contained in the medium of the present invention may be a saturated fatty acid or an unsaturated fatty acid.

The medium of the present invention can be prepared by, for example but not limited to, adding sodium hydrogen carbonate, selenium, transferrin, insulin, bFGF and 2-aminoethanol to a basal medium free of the selected neuronal differentiation associated amino acid (e.g., L-glutamine, asparagine or aspartic acid).

The pH of the medium of the present invention is preferably about 5.0 - about 8.5, and more preferably adjusted to about 6.0 - about 8.0. It is preferable to perform a sterilization treatment of the medium such as sterilization by filtration using a membrane filter and the like, and the like.

The form of the medium of the present invention is not particularly limited as long as the desired effects of the present invention can be achieved and it can be prepared in the form of, for example, liquid medium, semifluid medium or solid medium. In addition, the medium of the present invention may be prepared in the form of a powder. By preparing in the form of a powder, transportation and preservation may extremely facilitated. Such powder medium can be conveniently formed into a liquid medium by adding sterile water and the like immediately before use and sterilizing as necessary using membrane filter and the like. The medium of the present invention can be used for any of the culture methods such as adhesion culture, suspension culture, embedding culture, tissue culture and the like.

### (3) Culture method of neural stem cells and/or neural progenitor cells

The present invention provides a method for culturing neural stem cells and/or neural progenitor cells, comprising culturing the neural stem cells and/or the neural progenitor cells in the above-mentioned medium of the present invention (in the present specification, to be also referred to as the culture method of the present invention).

By culturing neural stem cells and/or neural progenitor cells in the medium of the present invention, neural stem cells and/or neural progenitor cells with promoted neuronal differentiation potency can be obtained. Particularly, when the neural stem cells and/or neural progenitor cells cultured in the medium of the present invention are differentiated into nerve cells, a nerve cell population with suppressed proliferation can be obtained. Thus, the culture method of the present invention is useful for producing nerve cells for nerve cell transplantation. Therefore, the culture method of the present invention is preferably a method for producing neural stem cells and/or neural progenitor cells with promoted neuronal differentiation potency. In addition, the neuronal differentiation potency of neural stem cells and/or neural progenitor cells can be promoted by reducing the concentration of one or more amino acids selected from the group consisting of L-glutamine, L-asparagine and L-aspartic acid in the medium in the maintenance culture of the neural stem cells and/or neural progenitor cells to a concentration at which the amino acid is "substantially absent" and continuously culturing the cells. The present invention can also be taken as a method for promoting the neuronal differentiation potency of such neural stem cells and/or neural progenitor cells. The concentration of the selected neuronal differentiation associated amino acid can be decreased by switching the medium used for culturing from a medium containing a given concentration of the selected neuronal differentiation associated amino acid to the medium of the present invention. By suspending a solution of cells suspended in a medium containing a neuronal differentiation associated amino acid in an about 10-fold amount of ΔGln medium, the medium before exchange is diluted about 10-fold. When a medium is exchanged by a general operation, carrying over of the medium before exchange is estimated to be about 1/100 volume at most. For example, by substituting the medium after the medium switching with the medium of the present invention immediately after or within a few hours from the medium switching, a medium containing a neuronal differentiation associated amino acid is estimated to be diluted at least about 1,000-fold. As mentioned above, a general medium for maintaining pluripotent stem cells or neural stem cells contains about 0.3 - 3.0 mM L-glutamine, about 0.05 - 1.0 mM L-asparagine and about 0.05 - 1.0 mM L-aspartic acid. Therefore, for example, neural stem cells and/or neural progenitor cells are maintenance cultured in a medium containing L-glutamine at a concentration of 0.3 - 3.0 mM, the medium is exchanged with the medium of the present invention substantially free of L-glutamine and the maintenance culture is continued, whereby the L-glutamine concentration is decreased from 0.3 - 3.0 mM to, for example, not more than 100 µM, preferably not more than 10 µM, more preferably not more than 100 nM, furthermore preferably not more than 1 nM, most preferably 0 nM, to promote neuronal differentiation potency of the neural stem cells and/or neural progenitor cells. In addition, for example, neural stem cells and/or neural progenitor cells are maintenance cultured in a medium containing L-asparagine at a concentration of 0.1 - 1.0 mM, the medium is exchanged with the medium of the present invention substantially free of L-asparagine and the maintenance culture is continued, whereby the L-asparagine concentration is decreased from 0.1 - 1.0 mM to, for example, not more than 10 µM, preferably not more than 100 nM, more preferably not more than 1 nM, most preferably 0 nM, to promote neuronal differentiation potency of the neural stem cells and/or neural progenitor cells. In addition, neural stem cells and/or neural progenitor cells are maintenance cultured in a medium containing L-aspartic acid at a concentration of 0.1 - 1.0 mM, the medium is exchanged with the medium of the present invention substantially free of L-aspartic acid and the maintenance culture is continued, whereby the L-aspartic acid concentration is decreased from 0.1 - 1.0 mM to, for example, not more than 10 µM, preferably not more than 100 nM, more preferably not more than 1 nM, most preferably 0 nM, to promote neuronal differentiation potency of the neural stem cells and/or neural progenitor cells.

The neural stem cells and/or neural progenitor cells to be used in the culture method of the present invention are preferably isolated. The "isolation" means that an operation to remove factors other than the object components or cells has been performed and they are out of their naturally occurring state. The purity of the "isolated neural stem cells and/or neural progenitor cells" (percentage of the number of neural stem cells and/or neural progenitor cells in the total number of cells) is generally not less than 70%, preferably not less than 80%, more preferably not less than 90%, further preferably not less than 99%, most preferably 100%.

The concentration of the neural stem cells and/or neural progenitor cells in the medium in the culture method of the present invention is not particularly limited as long as a desired effect is provided. It is generally 5x10³ - 10⁵ cells/cm³, preferably, 10⁴ - 3x10⁴ cells/cm³.

The culture conditions in the culture method of the present invention are not particularly limited as long as the desired effects can be achieved except that the medium of the present invention is used, and general culture conditions used for culturing neural stem cells and/or neural progenitor cells can be appropriately adopted according to the object of culture.

In the culture method of the present invention, the incubator used for cell culture is not particularly limited as long as a desired effect can be achieved. Examples thereof include flask, tissue culture flask, dish, petri dish, tissue culture dish, multidish, microplate, microwell plate, Multiplate, multiwell plate, microslide, chamber slide, schale, tube, tray, culture bag, and roller bottle and the like.

The incubator to be used for cell culture may be cell adhesive or cell non-adhesive, and is appropriately selected according to the object.

A cell adhesive incubator may be coated with any cell supporting substrate such as extracellular matrix (ECM) and the like or an artificial material mimicking the functions thereof, for the purpose of improving the adhesiveness to the cells on the surface of the incubator.

Other culture conditions can be appropriately set. For example, the culture temperature is not particularly limited as long as a desired effect can be achieved. It is about 30 - 40°C, preferably about 37°C. The CO₂ concentration is about 1 - 10%, preferably about 2 - 5%. The oxygen concentration is generally 1 - 40% and appropriately selected according to the culture conditions and the like.

In the culture method of the present invention, the neural stem cells and/or neural progenitor cells can be cultured by a method known per se such as adhesion culture, suspension culture, tissue culture and the like.

The period of culturing the neural stem cells and/or neural progenitor cells in the culture method of the present invention is generally not less than 2 days, preferably not less than 4 days, more preferably not less than 8 days, though it is not particularly limited as long as the desired effects of promotion of neuronal differentiation potency and the like can be achieved. The neural stem cells and/or neural progenitor cells cultured in the medium of the present invention are recovered, a part or all of them are passaged in a fresh medium of the present invention, and continuously cultured, whereby the neural stem cells and/or neural progenitor cells can be passaged while maintaining the promoted neuronal differentiation potency.

When neural stem cells and/or neural progenitor cells are passaged, neural stem cells and/or neural progenitor cells may be dispersion treated by a method known per se. Examples of the method for a dispersion treatment of cells include a treatment with a chelating agent (e.g., EDTA), an enzyme (e.g., trypsin, collagenase) and the like, an operation of mechanical dispersion (e.g., pipetting) and the like.

### (4) Production method of nerve cells from neural stem cells and/or neural progenitor cells

The present invention provides a production method of nerve cells characteristically including culturing neural stem cells and/or neural progenitor cells in the above-mentioned medium of the present invention, and continuously performing differentiation induction culture into nerve cells to give a nerve cell population (to be referred to as nerve cell production method of the present invention in the present specification).

In the present specification, a nerve cell population means a population of cells containing nerve cells. The nerve cell population may contain cells other than nerve cells. While the nerve cell population is not particularly limited as long as it includes nerve cells, generally not less than 1%, preferably not less than 5%, more preferably not less than 20%, further preferably not less than 40%, of the cells in the cell population are nerve cells.

Using the nerve cell production method of the present invention, many nerve cells can be produced in a short period with high efficiency. In addition, the nerve cell population obtained by the nerve cell production method of the present invention has a low risk of tumorigenesis when used for cell transplantation treatment since the nerve cell population has a low rate of proliferative cells. Therefore, the nerve cells produced by the nerve cell production method of the present invention can be preferably used for a transplantation treatment.

The nerve cell production method of the present invention includes the following steps:
(I) culturing a neural stem cell and/or a neural progenitor cell in the medium of the present invention to give the neural stem cell and/or the neural progenitor cell with promoted neuronal cell differentiation potency;
(II) culturing the neural stem cell and/or the neural progenitor cell with promoted neuronal differentiation potency obtained in step (I) under nerve cell differentiation induction conditions to give a nerve cell population.

Step (I) can be achieved by culturing neural stem cells and/or neural progenitor cells in the medium of the present invention for a period sufficient for promoting neuronal differentiation potency (e.g., not less than 2 days, preferably not less than 4 days, more preferably not less than 8 days). Other culture conditions are in accordance with the description of the culture method of the present invention.

The nerve cell differentiation induction conditions in step (II) are not particularly limited as long as neural stem cells and/or neural progenitor cells can be differentiated into nerve cells, and culture conditions used for differentiation induction into desired nerve cells can be adopted as appropriate. Examples of the adhesion culture method of neural stem cells and/or neural progenitor cells include the methods described in Flanagan LA et al., J Neurosci Res.2006 Apr; 83(5):845-56, Conti L et al., PLoS Biology., 2005 Sep; 3(9):e283 and the like. Suspension culture of neural stem cells and/or neural progenitor cells means culturing neural stem cells and/or neural progenitor cells in a medium under conditions non-adhesive to an incubator or feeder cells (when used). Examples of the suspension culture method of the neural stem cells and/or neural progenitor cells include neurosphere method (Reynolds BA and Weiss S., Science, USA, 1992 Mar 27; 255(5052):1707-10.), Serum-free Floating culture of Embryoid Body-like aggregates method (SFEB method, SFEBq method; Watanabe et al., Nature Neuroscience 8, 288-296(2005)) and the like. Tissue culture of neural stem cells and/or neural progenitor cells is a method of culturing a tissue containing neural stem cells and/or neural progenitor cells as a tissue section such as slice and the like or the whole tissue. Examples of the tissue culture of the neural stem cells and/or neural progenitor cells include the slice culture methods described in O'Rourke NA et al., Science. 1992 Oct 9; 258(5080):299-302., Komuro H et al., Science. 1992 Aug 7; 257(5071):806-9. and the like. The nerve cell differentiation induction conditions are well known to those of ordinary skill in the art, and those of ordinary skill in the art can appropriately adopt desired nerve cell differentiation induction conditions.

The concentration of the neural stem cells and/or neural progenitor cells in the medium in step (II) is not particularly limited as long as a desired effect is provided. It is generally 5x10³ - 10⁵ cells/cm³, preferably, 10⁴ - 3x10⁴ cells/cm³.

The culture conditions for the differentiation induction culture in step (II) are not particularly limited as long as desired effects such as reduced ratio of proliferative cells and the like are provided, and a known culture method can be appropriately adopted according to the object of culture.

In the differentiation induction culture in step (II), the incubator used for cell culture is not particularly limited as long as a desired effect can be achieved. Examples thereof include flask, tissue culture flask, dish, petri dish, tissue culture dish, multidish, microplate, microwell plate, Multiplate, multiwell plate, microslide, chamber slide, schale, tube, tray, culture bag, and roller bottle and the like.

The incubator used for cell culture may be cell adhesive or cell non-adhesive, and is appropriately chosen according to the object.

A cell adhesive incubator may be coated with any cell supporting substrate such as extracellular matrix (ECM) and the like or an artificial material mimicking the functions thereof, for the purpose of improving the adhesiveness to the cells on the surface of the incubator.

Other culture conditions can be appropriately set. For example, the culture temperature is not particularly limited as long as a desired effect can be achieved. It is about 30 - 40°C, preferably about 37°C. The CO₂ concentration is about 1 - 10%, preferably about 2 - 5%. The oxygen concentration is generally 1 - 40% and appropriately selected according to the culture conditions and the like.

In the differentiation induction culture in step (II), the neural stem cells and/or neural progenitor cells can be cultured by methods known per se such as adhesion culture, suspension culture, tissue culture and the like.

In the differentiation induction culture of step (II), while the period of culturing the neural stem cells and/or neural progenitor cells is not particularly limited as long as the desired effects such as production of nerve cells with decreased proliferation and the like can be achieved, culturing is continued at least until the nerve cell emerges. Emergence of nerve cell can be confirmed by evaluating the expression of the aforementioned nerve cell markers.

In one embodiment, differentiation induction culture includes culturing neural stem cells and/or neural progenitor cells with promoted neuronal differentiation potency in a differentiation induction medium.

While the period of culturing the neural stem cells and/or neural progenitor cells is not particularly limited as long as the desired effects such as production of nerve cells with decreased proliferation and the like can be achieved, when a differentiation induction medium is used for differentiation induction culture in step (II), it is generally not less than 10 days, preferably not less than 21 days, more preferably not less than 60 days. When culturing is performed in a differentiation induction medium, the medium is exchanged with a fresh medium generally once in 3 days, preferably not less than once in 2 days.

When a differentiation induction medium is used, it is preferable to perform a dispersion treatment of neural stem cells and/or neural progenitor cells in step (II) and subjecting the dispersion treated cells to differentiation induction culture. When a differentiation induction medium is used, a cell adhesive container is preferably used in step (II).

The composition of the differentiation induction medium used in step (II) is not particularly limited as long as differentiation of neural stem cells and/or neural progenitor cells into nerve cells can be achieved and a known medium for nerve cell differentiation induction can be used.

The differentiation induction medium preferably does not contain a substance maintaining an undifferentiated state of neural stem cells such as EGF, bFGF and the like.

As the differentiation induction medium to be used in step (II), a medium generally used for culturing mammalian cells may be prepared as a basal medium. The basal medium is not particularly limited as long as a desired effect can be achieved. For example, media usable for culturing animal cells such as BME medium, BGJb medium, CMRL 1066 medium, Glasgow MEM medium, Improved MEM Zinc Option medium, IMDM medium, Medium 199 medium, Eagle MEM medium, αMEM medium, DMEM medium, F-12 medium, DMEM/F12 medium, IMDM/F12 medium, ham medium, RPMI 1640 medium, Fischer's medium, or a mixed medium of these, and the like can be mentioned.

The differentiation induction medium used in step (II) is preferably a medium containing components determined chemically (Chemically defined medium; CDM) to avoid contamination with chemically-undefined components. The differentiation induction medium is preferably a serum-free medium to avoid contamination with chemically-undefined components. The "serum-free medium" in the present invention means a medium free of an unadjusted or unpurified serum. In the present invention, a medium containing purified blood-derived components or animal tissue-derived components is also included in the serum-free medium when an unadjusted or unpurified serum is absent.

The serum-free medium may contain a serum replacement. Examples of the serum replacement include those appropriately containing serum albumin, fatty acids, non-essential amino acids, transferrin, collagen precursor, trace element, 2-mercaptoethanol or 3'thiolglycerol, or equivalents of these and the like. Such serum replacement can be prepared by the method described in, for example, WO 98/30679. As a serum replacement, a commercially available product may also be used. Examples of the commercially available serum replacement include, but are not limited to, Knockout™ Serum Replacement (manufactured by Life Technologies: hereinafter sometimes to be also indicated as KSR), Glutamax™ (manufactured by Life Technologies), Chemically-defined Lipid concentrated (manufactured by Life Technologies), B27 (Life Technologies Inc.), N2 (Life Technologies Inc.).

The differentiation induction medium used in step (II) may further contain a medium additive. Examples of the medium additive include, but are not limited to, vitamins, proteins such as cytokines, growth factors and the like, L-ascorbic acid, ascorbyl-2-phosphate magnesium, sodium pyruvate, 2-aminoethanol, glucose, sodium hydrogen carbonate, HEPES, insulin, progesterone, sodium selenate, putrescine and the like. The additives are preferably added at a concentration within the range known per se.

The differentiation induction medium used in step (II) may contain L-glutamine, L-asparagine and L-aspartic acid.

The differentiation induction medium to be used in step (II) may contain a fatty acid. Examples of the fatty acid to be contained in the differentiation induction medium include, but are not limited to, oleic acid, linoleic acid, α-linolenic acid, γ-linolenic acid, palmitic acid, stearic acid, arachidonic acid, icosapentaenoic acid, docosahexaenoic acid, butyric acid, acetic acid, pulmitoleic acid, valeric acid, caproic acid, enanthic acid(heptylic acid), caprylic acid, pelargoric acid, capric acid, lauric acid, myristic acid, pentadecylic acid, margaric acid, khusenic acid, eleostearic acid, arachidic acid, 8,11-eicosadienoic acid, 5,8,11-eicosatrienoic, behenic acid, lignoceric acid, nervonic acid, cerotic acid, montanic acid, melissic acid and the like can be mentioned. The fatty acid contained in the differentiation induction medium may be a saturated fatty acid or an unsaturated fatty acid.

The pH of the differentiation induction medium is preferably about 5.0 - about 8.5, and more preferably adjusted to about 6.0 - about 8.0. It is preferable to perform a sterilization treatment of the medium such as sterilization by filtration using a membrane filter and the like, and the like.

In one embodiment of the present invention, the differentiation induction medium contains DMEM/F12 medium as a basal medium and containing transferrin, Glutamax, B27 supplement, N2 supplement, glucose, sodium hydrogen carbonate, HEPES, insulin, progesterone, sodium selenite and putrescine.

When the above-mentioned medium is used, while the period of culturing the cells in step (II) is not particularly limited as long as differentiation induction into nerve cell can be achieved, it is generally not less than 10 days, preferably not less than 21 days, more preferably not less than 60 days.

Step (II) may cause differentiation into a particular kind of nerve cell.

The nerve cell production method of the present invention may further include step (III) of isolating nerve cells from the nerve cell population continuously from steps (I) and (II).

Isolation of nerve cells from the nerve cell population can be performed by a method known per se.

Examples of the method of isolating nerve cells from the nerve cell population include, but are not limited to, an isolation method by flow cytometry using an antibody against an extracellular antigen specifically present in the desired nerve cells, an isolation method using microbeads bound with an antibody against an extracellular antigen specifically present in the desired nerve cells and the like.

Isolation of nerve cell population can also be achieved by removing neural progenitor cells and neural stem cells predicted to cause contamination.

### (5) Neural stem cells and/or neural progenitor cells with promoted neuronal differentiation potency

The present invention provides neural stem cells and/or neural progenitor cells obtained by the above-mentioned culture method of the present invention (neural stem cells and/or neural progenitor cells of the present invention).

As mentioned above, the neural stem cells and/or neural progenitor cells of the present invention have promoted neuronal differentiation potency.

The neural stem cells and/or neural progenitor cells of the present invention can be obtained by culturing neural stem cells and/or neural progenitor cells in the medium of the present invention generally for not less than 2 days, preferably not less than 4 days, more preferably not less than 8 days. Other culture conditions are in accordance with the description of the culture method of the present invention.

The neural stem cells and/or neural progenitor cells of the present invention are preferably isolated. The "isolation" means that an operation to remove factors other than the object components or cells has been performed and they are out of their naturally occurring state. The purity of the "isolated neural stem cells and/or neural progenitor cells" (percentage of the number of neural stem cells and/or neural progenitor cells in the total number of cells) is generally not less than 70%, preferably not less than 80%, more preferably not less than 90%, further preferably not less than 99%, most preferably 100%.

As one embodiment, the neural stem cells and/or neural progenitor cells of the present invention can be provided in a cryopreserved state. The neural stem cells and/or neural progenitor cells of the present invention can be cryopreserved, and can be used by melting and initiating as necessary. For cryopreservation, a cell cryopreservation method known per se can be used. As an example of cryopreservation, dimethylsulfoxide is added to the composition of the present invention and the neural stem cells and/or neural progenitor cells of the present invention are preserved under the conditions of -80 to -200°C, preferably -196°C (in liquid nitrogen).

### (6) Culture preparation of neural stem cells and/or neural progenitor cells

The present invention provides a culture preparation of neural stem cells and/or neural progenitor cells containing the above-mentioned medium and neural stem cells and/or neural progenitor cells of the present invention (culture preparation of the present invention).

The neural stem cells and/or neural progenitor cells in the culture preparation of the present invention are viable and proliferating cells.

The neural stem cells and/or neural progenitor cells in the culture preparation of the present invention are preferably isolated. The "isolation" means that an operation to remove factors other than the object components or cells has been performed and they are out of their naturally occurring state. The purity of the "isolated neural stem cells and/or neural progenitor cells" (percentage of the number of neural stem cells and/or neural progenitor cells in the total number of cells) is generally not less than 70%, preferably not less than 80%, more preferably not less than 90%, further preferably not less than 99%, most preferably 100%.

In the culture preparation of the present invention, the neural stem cells and/or neural progenitor cells are present in the medium of the present invention. In one embodiment, the culture preparation of the present invention is a suspension of the neural stem cells and/or neural progenitor cells in the medium of the present invention. The culture preparation of the present invention may be encapsulated in an appropriate container.

The culture preparation of the present invention is useful for performing the above-mentioned culture method of the present invention.

In one embodiment, the culture preparation of the present invention can be provided in a cryopreserved state. The culture preparation of the present invention can be cryopreserved, and can be used by melting and initiating as necessary. For cryopreservation, a cell cryopreservation method known per se can be used. As an example of cryopreservation, dimethyl sulfoxide is added to the culture preparation of the present invention and the culture preparation of the present invention is preserved under the conditions of -80 to -200°C, preferably -196°C (in liquid nitrogen).

The present invention is explained in more detail in the following by referring to Examples, which are not to be construed as limitative.

### [Example]

### Example 1: Culture method of neural stem cells in medium substantially free of glutamine

### (1) Induction method of long-term self-renewing neuro epithelial-like stem cells (hereinafter LtNES cells)

EB (embryoid body) was formed from iPS cells, and cultured for 4 days in a medium corresponding to the composition of E6 medium (Life Technologies or STEMCELL Technologies) from which ascorbic acid and transferrin were removed and to which transferrin (final concentration 0.5 - 10 µg/mL), ethanolamine (final concentration 5 - 50 µM), human serum albumin (final concentration 0.1 - 5 mg/mL) were added. EB was seeded into a dish coated with poly-L-ornithine (PO), cultured in the above-mentioned medium for about 10 days and formation of a Rosette-like structure was confirmed. The Rosette part was hollowed out, and suspension cultured as a neurosphere in the above-mentioned medium for about 7 days. Neurosphere was dispersed using trypsin/EDTA and cultured in an RHB-A medium in PO/laminin-coated dish to prepare LtNES cells.

### (2) Culture method of LtNES cells

To a medium corresponding to the composition of E6 medium (Life Technologies or STEMCELL Technologies) from which ascorbic acid, transferrin and L-glutamine were removed were added transferrin (final concentration 0.5 - 10 µg/mL), ethanolamine (final concentration 5 - 50 µM), human serum albumin (final concentration 0.1 - 5 mg/mL) and 20 ng/mL bFGF to give an L-glutamine-free medium (ΔGln medium). In addition, a control medium (Full medium) having the same composition as the ΔGln medium except that L-glutamine (0.3-3.0 mM) was contained was prepared.

LtNES cells induced from human pluripotent stem cells were suspended in the Full medium to give a cell suspension. The cell suspension was added in a 1/10 amount to the Full medium or ΔGln medium and the mixture was cultured under 37°C, 5% CO₂ environment. To reduce carrying over of the medium before exchange, the medium was exchanged 2 hr after the start of the culturing, and culturing was continued. Therefore, the medium before exchange is estimated to be diluted at least about 1,000-fold. For passaging of LtNES cells, the cells were incubated in TrypLE Select at 37°C for 1 min, diluted with the medium, and single cells were obtained by pipetting. The cells dispersed in the above-mentioned medium at 1.5x10⁵ cells/well (6 well plate) were seeded and cultured under 37°C, 5% CO₂ environment. The cell count was measured by blood cell counting after dead cell staining with Trypan Blue (Life Technologies).

A microscopic image of the neural stem cells at 4 days of culture is shown in Fig. 1A. The neural stem cells cultured for 4 days in the ΔGln medium showed a form similar to that of the neural stem cells cultured in the Full medium.

### Example 2: Verification of promoting effect on neuronal cell induction

To verify differentiation potency of cells cultured in an L-glutamine-free medium, a differentiation induction experiment was performed.

In the same manner as in Example 1, neural stem cells were induced from iPS cells, and cultured in an L-glutamine-free medium (ΔGln medium) or control medium (Full medium) for 4 days.

After culturing, TrypLE Select was added instead of the medium, the cells were incubated at 37°C for 1 min and subjected to a cell dispersion treatment. TrypLE Select was diluted with the medium, pipetting was performed, and the cells were seeded in a poly-L-ornithine/fibronectin-coated 48 well plate at 1.5x10⁵ cells/well. The cells were cultured for 21 days from Day4 to Day25 in a differentiation induction medium obtained by adding 1xN2 supplement (Life Technologies) and 1xB27 supplement (Life Technologies) to DMEM/F-12 (Life Technologies) containing L-glutamine. The cells were cultured in an incubator under 37°C, 5% CO₂ atmosphere. The medium was exchanged once every two days.

RNA was extracted from the cells after differentiation induction culture, transcribed to cDNA, qPCR was performed, and the expression intensity of Neuro D1 (Neurogenic differentiation 1), synapsin 1 was measured. The cells after differentiation induction culture were fixed and immunostaining was performed by a fluorescent antibody method using antibody against a nerve cell marker βIII tubulin.

The results of the immunostaining are shown in Fig. 1C. The cells after differentiation induction were shown to contain βIII tubulin-positive differentiated nerve cells. The neural stem cells cultured in the ΔGln medium produced more differentiated nerve cells than the neural stem cells cultured in the Full medium. Therefore, the ΔGln medium was shown to have a promoting action on neural stem cell differentiation.

The results of qPCR are shown in Fig. 1D. The expression of a nerve cell marker Synapsin1 and a neural progenitor cell marker NeuroD1 was promoted in the nerve cells induced from the neural stem cells cultured in the ΔGln medium for L-glutamine.

### Example 3: Verification of suppressive effect on nerve cell proliferation

According to the neural stem cell culture method described in Example 1, neural stem cells were cultured for 4 days in a control medium (Full medium) or L-glutamine-free medium (ΔGln medium). The cultured cells were fixed with 4%-para-formaldehyde phosphate buffer at room temperature for 20 min. The fixed cells were washed with PBS (Phosphate buffered saline), incubated in ethanol at room temperature for 5 min and air-dried. Thereafter, the cells were stained with 0.4% crystal violet methanol solution (Wako Pure Chemical Industries, Ltd.: 038-04862) for 20 min and washed with pure water. 1% aqueous SDS solution (350 µL) was added and the mixture was shaken for 20 min to elute crystal violet and the absorbance of the eluate at 570 nm was measured by a microplate reader SH-9000 (CORONA ELECTRIC Co., Ltd.), based on which the cell number was quantified.

The results are shown in Fig. 1B. The number of neural stem cells cultured in the ΔGln medium for 4 days was less than that when cultured in the Full medium. Therefore, it was shown that the ΔGln medium suppresses proliferation of neural stem cells.

### Example 4: Verification of proliferation effect on neural stem cells

According to the neural stem cell culture method described in Example 1, neural stem cells were cultured for 4 days in a control medium (Full medium) or L-glutamine-free medium (ΔGln medium). Culturing was continued until day 20 (total 5 passages), and the cell number was counted on days 4, 8, 12, 16 and 20 of culture.

The results are shown in Fig. 1E. The number of neural stem cells cultured in the ΔGln medium showed linear proliferation (Fig. 1E).

### Example 5: Analysis of amino acids in medium

According to the neural stem cell culture method described in Example 1, neural stem cells were cultured for not less than 4 days in a control medium (Full medium) or L-glutamine-free medium (ΔGln medium). The medium was removed and the cultured cells were dissolved in ice-cold methanol and recovered. The amount of amino acids in the cell, and the amounts of keto acids and organic acids relating to TCA cycle were analyzed. The analysis procedures of amino acids, keto acids and organic acids are shown below.

### <Analysis procedure of amino acids>

### Derivatizing reaction of sample solution

A sample (10 µL) was collected, an internal standard solution (10 µL) was added, borate buffer (60 µL) for aminotag Wako APDS tag Wako (manufactured by Wako Pure Chemical Industries, Ltd.) was added and the mixture was shaken. Furthermore, an aminotag Wakoamino acid analysis reagent (for LC/MS) (manufactured by Wako Pure Chemical Industries, Ltd.) (20 µL) was added and the mixture was vigorously shaken.

Using a block heater, the mixture was thereafter heated at 55°C for 10 min. After cooling, a dilution solution (400 µL) was added to the reaction solution and the mixture was shaken to give an analysis sample.

### Analysis of derivatizing reaction solution

Using, as the mobile phase, an eluent for aminotag Wako APDS tag Wako (manufactured by Wako Pure Chemical Industries, Ltd.) as SOLUTION A and a mixture of acetonitrile (600 µL) and water (400 µL) as SOLUTION B, and C8 column as the stationary phase, the sample was introduced into LC-MS/MS system and detected by the mass number of each amino acid.

As the internal standard, an amino acid internal standard mixture for aminotag Wako APDS tag Wako (manufactured by Wako Pure Chemical Industries, Ltd.), No. 1 (650 µl) was collected, No. 2 (50 µL) was added, and a mixture of 50 µM Put-d8 and water at a ratio of 1:1:8 was used.

### Analysis apparatus

As the analysis apparatus, API4000 LC/MS/MS system (manufactured by AB SCIEX) was used.

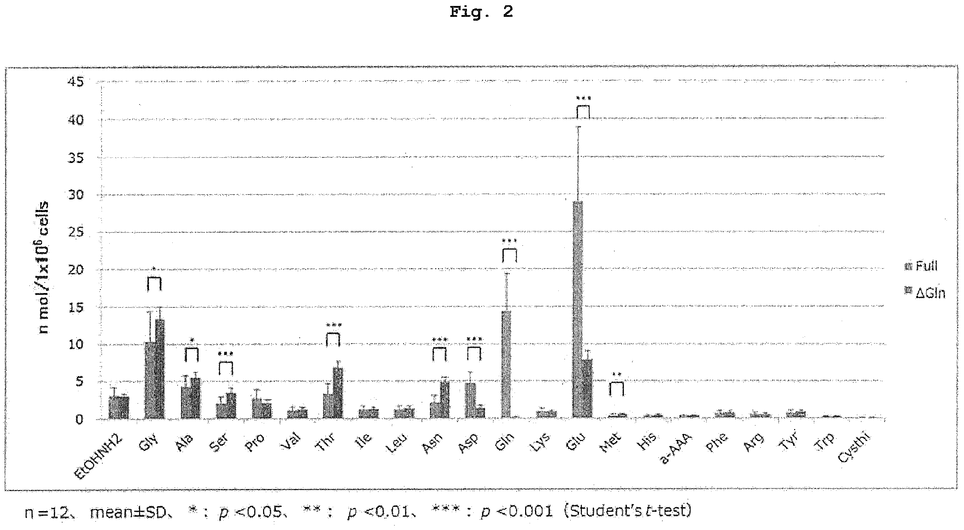

The results are shown in Fig. 2. The intracellular amino acid content of the cells cultured in the ΔGln medium changed, and a significant increase was observed in glycine, alanine, serine, threonine and asparagine. In addition, a significant decrease was observed in glutamine, glutamic acid and aspartic acid.

### <Analysis procedure of keto acids>

### Preparation of derivatizing reagent solution

O-(2,3,4,5,6-pentafluorobenzyl)-hydroxylamine (10 mg) was dissolved in a solution (1 mL) of a mixture of equal amounts of 0.1% aqueous sodium hydroxide solution and acetonitrile to give a derivatizing reagent solution.

### Derivatizing reaction of sample solution

A sample solution (20 µL) was placed in a reaction vial, a derivatizing reagent solution (20 µL) was added and the mixture was stirred well. The mixture was stood in ice water for 30 min to perform a derivatizing reaction. Thereafter, a solution (10 µL) of a mixture of equal amounts of acetone and acetonitrile was added to discontinue the derivatizing reaction. Thereafter, a solution (50 µL) of a mixture of equal amounts of 0.1% aqueous sodium hydroxide solution and acetonitrile was added and the diluted solution was used as an analysis sample.

### Analysis of derivatizing reaction solution

Using, as the mobile phase, a 25 mM aqueous formic acid solution as SOLUTION A and acetonitrile as SOLUTION B, and Ph column as the stationary phase, the analysis sample was introduced into LC-MS/MS system and detected by the mass number corresponding to each keto acid.

### Analysis apparatus

As the analysis apparatus, API4000 LC/MS/MS system (manufactured by AB SCIEX) was used.

### <Quantification procedure of organic acids>

### Preparation of derivatizing reagent solution

3-Picolylamine (10 mg) was dissolved in a DMSO solution containing 5% triethylamine to 200 mM to give a derivatizing reagent solution.

### Preparation of condensing agent solution

4-(4,6-Dimethoxy-1,3,5-triazin-2-yl)-4-methylmorpholinium hydrochloride was dissolved in DMSO to 50 mM to give a condensing agent solution.

### Preparation of internal standard solution

A stable isotope-labeled compound of each organic acid was dissolved in distilled water to a concentration of 100 µM to give an internal standard stock solution. Equal amounts of respective internal standard stock solutions were separated, and distilled water was added to a final concentration of 10 µM to give an internal standard solution.

### Derivatizing reaction of sample solution

A sample solution (100 µL) was placed in a reaction vial, the internal standard solution (10 µL) was added and the mixture was stirred well and dried under reduced pressure in a rotary evaporator. To the sample dried under reduced pressure were added 10 µL of the 50% acetonitrile solution, 15 µL of the derivatizing reagent solution and 30 µL of the condensing agent solution, and the sample was dissolved and heated at 80°C for 60 min to perform derivatization. The reaction solution (30 µL) was separated in a vial, a 50 mM aqueous ammonium formate solution (120 µL) was added, and the diluted solution was used as an analysis sample.

### Analysis of derivatizing reaction solution

Using, as the mobile phase, a 25 mM aqueous formic acid solution adjusted to pH 6 as SOLUTION A and acetonitrile as SOLUTION B, and C8 column as the stationary phase, the analysis sample was introduced into LC-MS/MS system and detected by the mass number corresponding to each organic acid.

### Analysis apparatus

As the analysis apparatus, API4000 LC/MS/MS system (manufactured by AB SCIEX) was used.

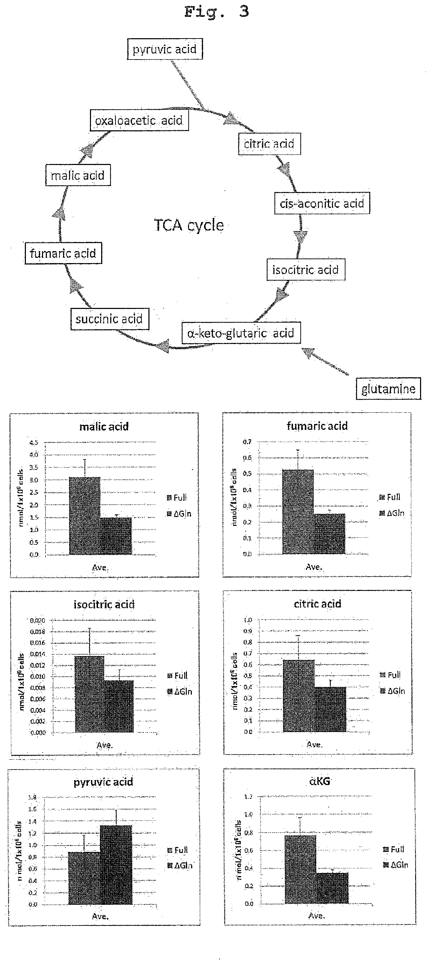

The results are shown in Fig. 3. The organic amino acid content, which relates to the intracellular TCA cycle, of the cells cultured in the ΔGln medium changed, and a significant increase was observed in pyruvic acid. In addition, a significant decrease was observed in α-ketoglutaric acid, citric acid, isocitric acid, fumaric acid and malic acid.

### Example 6: Verification of presence or absence of cell death of L-glutamine-removed medium cultured cells

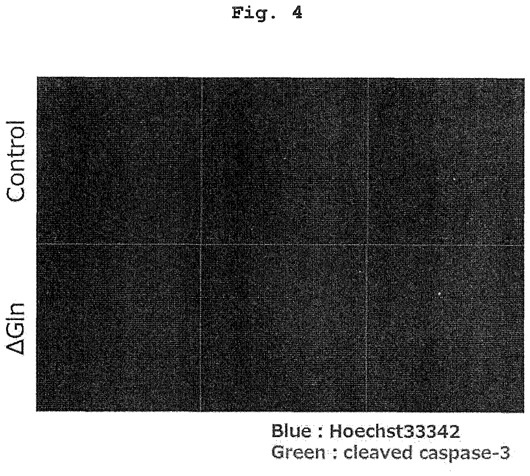

According to the neural stem cell culture method described in Example 1, neural stem cells were cultured for 4 days in a control medium (Full medium) or L-glutamine-free medium (ΔGln medium). The cultured cells were fixed with 4%-para-formaldehyde phosphate buffer at room temperature for 20 min. The fixed cells were washed with PBS, and immunostaining was performed using antibody against an apoptosis marker, cleaved caspase-3, and a nuclear staining agent, Hoechst33342. The results are shown in Fig. 4.

Apoptosis was hardly observed in the neural stem cells cultured in the L-glutamine-free medium (ΔGln) or the neural stem cells cultured in the L-glutamine-containing medium (Control). Apoptosis hardly occurred irrespective of the presence or absence of L-glutamine in the medium. Therefore, it was shown that cell death of some cells having high sensitivity does not occur in the L-glutamine-free medium.

### Example 7: Verification of promoting effect on neuronal differentiation by each non-essential amino acid-removed medium

A medium having the composition of the Full medium described in Example 1, from which each essential amino acid (glutamine, asparagine, aspartic acid, glutamic acid, alanine, glycine, serine or proline) was removed, was produced. In the same manner as in Example 1, neural stem cells were induced from iPS cells, and cultured in each non-essential amino acid-removed medium or Full medium for 8-12 days.

After culturing, TrypLE Select was added instead of the medium, the cells were incubated at 37°C for 1 min and subjected to a cell dispersion treatment. TrypLE Select was diluted with the medium, pipetting was performed, and the cells were seeded in a poly-L-ornithine/fibronectin-coated 48 well plate at 1.5x10⁵ cells/well. The cells were cultured for 20 days in a medium obtained by adding 1xN2 supplement (Life Technologies) and 1xB27 supplement (Life Technologies) to DMEM/F-12 (Life Technologies). The cells were cultured in an incubator under 37°C, 5% CO₂ atmosphere. The medium was exchanged once every two days.

The cells after culturing were fixed and immunostaining was performed by fluorescent antibody method using antibody against a nerve cell marker βIII tubulin. The neurite length of the βIII tubulin-positive cells was quantified by image analysis. The results are shown in Table 1. In Table 1, the symbols show relative average neurite lengths with the length of the control (Full) as 1, ++:>1.5, +:1.5 - 0.3, -:0.3>. The medium free of glutamine, the medium free of aspartic acid, and the medium free of asparagine showed an action to promote neuronal differentiation potency of neural stem cells.

**[Table 1]**

| medium | Evaluation of neurite length of βIII tubulin-positive cells |
|---|---|
| Control (full) | + |
| ΔGln | ++ |
| ΔAsn | ++ |
| ΔAsp | ++ |
| ΔGlu | + |
| ΔAla | - |
| ΔGly | + |
| ΔSer | + |
| ΔPro | + |

### [Industrial Applicability]

According to the present invention, the neuronal differentiation potency of neural stem cells and/or neural progenitor cells can be promoted, and human cost and monetary cost necessary for culturing neural stem cells and/or neural progenitor cells can be reduced.

This application is based on patent application No. 2016-071967 filed in Japan (filing date: March 31, 2016), the contents of which are encompassed in full herein.

## Claims

1. A medium substantially free of L-glutamine, for culturing neural stem cells and/or neural progenitor cells.

2. The medium according to claim 1, having an L-glutamine concentration of not more than 100 µM.

3. The medium according to claim 1 or 2, not comprising L-glutamine.

4. The medium according to any one of claims 1 to 3, comprising holotransferrin.

5. The medium according to any one of claims 1 to 4, comprising L-tryptophan, L-lysine, L-methionine, L-phenylalanine, L-threonine, L-valine, L-leucine, L-isoleucine and L-histidine.

6. The medium according to any one of claims 1 to 5 for promoting the neuronal differentiation potency of a neural stem cell and/or a neural progenitor cell.

7. The medium according to any one of claims 1 to 6 that is a serum-free medium.

8. A method for culturing a neural stem cell and/or a neural progenitor cell, comprising culturing the cell(s) in the medium according to any one of claims 1 to 7.

9. The method according to claim 8 for producing a neural stem cell and/or a neural progenitor cell with promoted neuronal differentiation potency.

10. The method according to claim 8 or 9, wherein the culturing is performed for a period of not less than 4 days.

11. A method for producing a nerve cell, comprising the following steps:
(I) culturing a neural stem cell and/or a neural progenitor cell in the medium according to any one of claims 1 to 7 to give the neural stem cell and/or the neural progenitor cell with promoted neuronal differentiation potency;
(II) culturing the neural stem cell and/or the neural progenitor cell with promoted neuronal differentiation potency and obtained in step (I) under nerve cell differentiation induction conditions to give a nerve cell population.

12. A neural stem cell and/or a neural progenitor cell with promoted neuronal differentiation potency and obtained by the method according to claim 9.

13. A culture preparation comprising a neural stem cell and/or a neural progenitor cell, and the medium according to any one of claims 1 to 7.
